# EUROPEAN PATENT SPECIFICATION

(11) **EP 0 901 392 B1**
(45) Date of publication and mention of the grant of the patent: **31.03.2004**
(21) Application number: 97925640.1
(22) Date of filing: 19.05.1997
(51) Int. Cl.: A61M 25/01

(54) **CATHETER BALLOON WITH AN EXTENDING CORE WIRE**
BALLONKATHETER MIT ERWEITENDEM KERNDRAHT
CATHETER A BALLONNET AVEC EXTENSION FILAIRE

(30) Priority: 20.05.1996 US 650464; 06.03.1997 US 813024
(43) Date of publication of application: 17.03.1999
(73) Proprietor: Medtronic AVE, Inc., Santa Rosa, California 95403 (US)
(72) Inventor: BAGAOISAN, Celso, J., Union City, CA 94587 (US); MUNI, Ketan, P., San Jose, CA 95136 (US); ZADNO-AZIZI, Gholam-Reza, Neward, CA 94560 (US)
(74) Representative: Van Malderen, Joelle
(86) International application number: PCT/US1997/008468
(87) International publication number: WO 1997/044084

(56) References cited:
- EP-A- 0 442 480
- EP-A- 0 631 792
- WO-A-92/00775
- WO-A-96/07351
- WO-A-96/13295
- WO-A-97/11735
- US-A- 4 838 268
- US-A- 5 167 239
- US-A- 5 211 636

## Description

### Background of the Invention

The present invention generally relates to medical devices, and in particular, to improved medical catheters featuring a core wire at the distal end of the catheter, like those disclosed in EP 0 442 480.

Medical catheters, such as occlusion balloon catheters and balloon dilatation catheters, have been proven efficacious in treating a wide variety of blood vessel disorders. Moreover, these types of catheters have permitted clinicians to treat disorders with minimally invasive procedures that, in the past, would have required complex and perhaps life threatening surgeries. For example, balloon angioplasty is now a common procedure to alleviate stenotic lesions (i.e., clogged arteries) in blood vessels, thereby reducing the need for heart bypass operations.

Because medical catheters must be passed through a tortuous blood vessel network to reach the intended treatment site, it is desirable that the catheters be fairly flexible, especially at the distal end. However, the distal end must not be so flexible that it tends to bend back upon itself when the clinician advances the catheter distal end through the patient.

One method of imparting desired flexibility characteristics to a catheter has been to incorporate a "core wire" into the distal end of the catheter. A core wire is a wire that extends through the catheter tubular body, providing structural support to the distal end to prevent bend backs during catheter advancement. Furthermore, the core wire is also flexible, such that the catheter distal end may navigate tortuous blood vessel networks.

Previously known catheter core wires are of complex construction, requiring multiple manufacturing steps to incorporate the core wire into the catheter. This increases manufacturing costs of the catheter, which ultimately are passed on to hospitals and patients. Moreover, previously known core wires may not be sufficiently flexible. Accordingly, there exists a need for catheter core wires that are easier to manufacture, and which possess the desired flexibility profiles.

### Summary of the Invention

The present invention provides a catheter core wire with improved flexibility and a simple and easily manufacturable design. In one aspect of the present invention, there is provided a catheter with a tubular body having a proximal end and a distal end. The tubular body has a lumen extending therethrough. An expandable member is mounted on the distal end of the tubular body. The expandable member has a proximal portion and a distal portion which are both mounted to the tubular body.

A core wire is inserted into the lumen at the distal end. The core wire has an end mounted within the distal end of the lumen and an extending portion which extends from the distal end of the tubular body. In one aspect of the invention, the extending portion is tapered through a length of no more than 60 mm but at least 5 mm, preferably 60 to 15 mm, more preferably 50 to 15 mm, and optionally 35 to 15 mm.

In one preferred embodiment, the core wire is tapered over a length of no more than 40 mm but at least 10 mm, and is made of a nitinol allay or stainless steel. The core wire may have a first cross-sectional area at one end of the taper, and a second cross-sectional area at the other end of the taper, the first cross-sectional area being greater than the second cross-sectional area by at least 20%. In another embodiment, the first cross-sectional area is greater than the second cross-sectional area by at least 70%. In these embodiments, the extending portion may also have a region of constant cross-sectional area.

In another aspect of the present invention, there is provided a hollow guidewire formed from a nitinol hypotube having a proximal end and a distal end. The proximal end has a first wall thickness and the distal end has a second wall thickness. The first wall thickness is greater than the second wall thickness. An expandable member is mounted on the distal end of the hypotube, and there is a tapered core wire extending from the distal end of the hypotube. In one embodiment, the first wall thickness is 20% greater than the second wall thickness.

In another aspect of the present invention, there is provided a hollow guidewire, formed of a nitinol hypotube having a proximal end and a distal end. The nitinol hypotube has a lumen extending between the proximal and distal ends. An expandable member is mounted on the distal end. A core wire is inserted into the lumen at the distal end of the nitinol hypotube, and the distal end is crimped on the core wire to secure it within the lumen.

There is disclosed a catheter having a tubular body. The tubular body has a proximal end and a distal end, and an irrigation lumen extending therethrough. An irrigation opening is on the distal end of the tubular body. The irrigation opening is in fluid communication with the irrigation lumen. A core wire has an end mounted within the lumen. The core wire has an extending portion which extends from the distal end of the tubular body, the extending portion being tapered through a length of no more than 60 mm but at least 5 mm.

### Brief Description of the Drawings

Figure 1 is a side view of a catheter incorporating the core wire of the present invention.
Figure 2 is a cross-sectional view along lines 2-2 of Figure 1.
Figure 3 is a cross-sectional view along lines 3-3 of Figure 1.
Figure 4 is a longitudinal cross-sectional view of the distal end of a catheter having the core wire of the present invention.

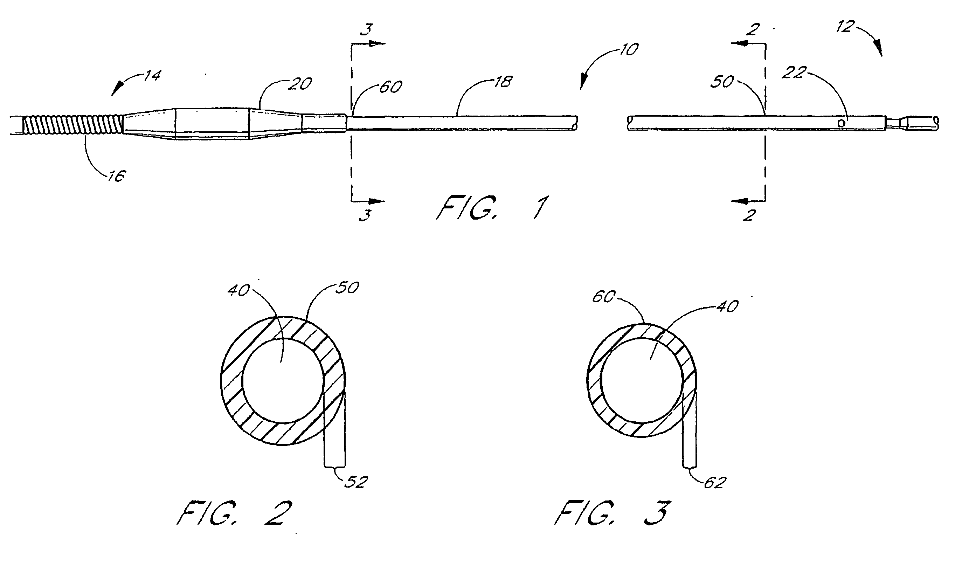

In the embodiment illustrated in Figure 1, the core wire of the present invention is incorporated in an occlusion balloon catheter 10. Catheter 10 generally comprises an elongate flexible tubular body 18 extending between a proximal control end 12 and a distal functional end 14. Tubular body 18 has a central lumen 40 which extends between ends 12 and 14. An inflation port 22 is provided on tubular body 18 near the proximal end. Inflation port 22 is in fluid communication with lumen 40, such that fluid passing through inflation port 22 into or out of lumen 40 may be used to inflate or deflate inflatable balloons in communication with lumen 40. Lumen 40 is seated fluid tight at distal end 14. Inflation port 22 may be similar to existing female luer lock adapters or could be a removable valve at the end.

The length of tubular body 18 may be varied considerably depending upon the desired application. For example, where catheter 10 serves as a guidewire for other catheters in a conventional percutaneous transluminal coronary angioplasty procedure involving femoral artery access, tubular body 18 is comprised of hollow hypotube having a length in the range of from about 160 to about 320 centimeters with a length of about 180 centimeters being optimal for a single operator device and 300 centimeters for over the wire applications. Alternately, for a different treatment procedure, not requiring as long a length of tubular body 18, shorter lengths of tubular body 18 may be provided.

Tubular body 18 generally has circular cross-sectional configuration with an outer diameter within the range of from about 0,2 to 3,5 mm (0.008 inches to 0.14 inches). In many applications where catheter 10 is to be used as a guidewire for other catheters, the outer diameter of tubular body 18 ranges from 0,25 to 0,9 mm (0.010 inches to 0.038 inches) and preferably is 0,45 mm (0.018 inches) in outer diameter or smaller. Non-circular cross-sectional configurations of lumen 40 can also be adapted for use with the present invention. For example, a triangular rectangular, oval, and other non-cucular cross-sectional configurations are also easily incorporated for use with present invention, as will be appreciated by those of skill in the art.

Tubular body 18 has sufficient structural integrity, or "pushability," to permit catheter 10 to be advanced through vasculature to distal arterial locations without buckling or undesirable kinking of tubular body 18. It is also desirable for tubular body 18 to have the ability to transmit torque, such as in those embodiments where it may be desirable to rotate tubular body 18 after insertion into a patient. The desired properties of structural integrity and torque transmission are achieved by forming tubular body 18 out of an alloy of titanium and nickel, commonly referred to as nitinol. In a more preferred embodiment, the nitinol alloy used to form tubular body 18 is comprised of about 50.8 % nickel and the balance titanium, which is sold under the trade name Tinel (TM) by Memry Corporation. It has been found that a catheter tubular body having this composition of nickel and titanium exhibits an improved combination of flexibility and kink resistance in comparison to other materials.

As shown in Figures 1·3, tubular body 18 may be formed of a hollow nitinol hypotube. Hollow nitinol hypotube 18 has a proximal portion 50 having a first wall thickness 52 and a distal portion 60 having a second wall thickness 62. Wall thickness 52 is at least 5% greater, preferably at least 20% greater, more preferably at least 40% greater, and may be as much as 60% greater or more than wall thickness 62. For example, where proximal portion 50 has a wall thickness of 0,05 mm (.002"), distal portion 60 has a wall thickness of 0,03 mm (.0013). The wall thickness may be reduced at the distal end of the tubular body from points starting about 30 cm proximal of balloon 20 to points just distal to the balloon. For example, wall thickness may be reduced starting at points 1 cm, 5 cm, 10 cm, 20 cm or 30 cm proximal of balloon 20. Alternatively, wall thickening may be reduced starting at a point just distal to balloon 20.

In one embodiment, the wall thickness is reduced by removing wall material from the outer diameter of the tubular body, while maintaining lumen diameter 40 constant, so as to introduce a gradual taper in the tubular body. The wall thickness of the nitinol tubular body may be reduced by any means known to those of skill in the art, such as grinding, swaging, or etching.

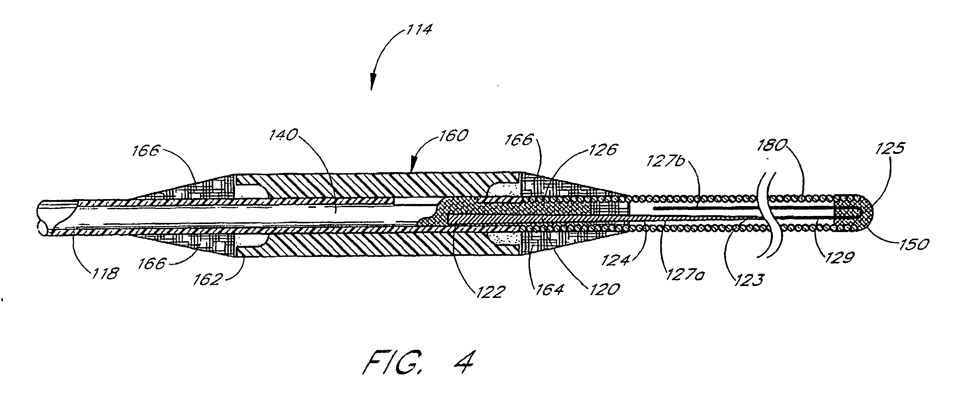

Referring to Figure 4, there is depicted a catheter distal end 114. Distal end 114 is provided with a tapering core wire 120 at the distal end of a tubular body 118. Tubular body 118 may have differing wall thicknesses along its length, as described previously. Core wire 120 is preferably formed of a shape memory alloy, such as nitinol, but may also be formed of other materials, such as stainless steel. A proximal end 122 of core wire 120 is inserted into a lumen 140 of tubular body 118 and is attached thereto. End 122 may be secured to lumen 140 by any means known to those of skill in the art, such as adhesives. Particularly preferred adhesives for attachment are cyanoacrylates of the type sold under the trade name Loctite™. Other adhesives, such as metal to metal bond adhesives may also be used. Core wire end 122 may also be secured within lumen 140 by welding or soldering.

Alternately, in another preferred embodiment, proximal end 122 of core wire 120 may be secured within lumen 140 by crimping tubular body 118 such that the interior surface of tubular body 118 defining lumen 140 contacts proximal end 122 and firmly secures it within lumen 140. Preferably, tubular body 118 is crimped at least two points, and more preferably at three or more points, to secure proximal end 122 within lumen 140. In those embodiments where tubular body 118 is made of nitinol, sufficient crimping pressure must be exerted upon tubular body 118 to overcome the elastic response of nitinol. Generally, this requires exertion of sufficient pressure to deform the nitinol tubular body 118 by 9 % or more. For a nitinol tubular body 118 having an outer diameter of 0,35 mm (0.014 inches) and an inner diameter of 0,34 mm (0.0095 inches) to be crimped over a nitinol core wire end 122 having an outer diameter of 0,2 mm (0.009 inches), it has been found that a pressure of 120 ksi is sufficient. Other pressures may also be used, provided that they are sufficient to cause tubular body 118 to securely contact core wire 122, but not so great as to unduly deform tubular body 118.

Core wire 120 may range in length from about 20 mm to about 100 mm or more, preferably from 25 mm to 50 mm, and, for most occlusive device applications, is typically about 40 mm. Extending portion 124 may have a length which varies from about 15 mm to about 95 mm or more, preferably 20 mm to 45 mm, and optimally about 35 mm.

Core wire 120 has a portion 124 which extends from tubular body 118. Extending portion 124 tapers from a larger cross-sectional diameter to a smaller cross-sectional diameter. Preferably, substantial all of the tapering of core wire 120 occurs in extending portion 124. In one preferred embodiment, the cross-sectional area of extending portion 124 decreases by at least 20%, preferably by 60%, more preferably by 70%, and optimally by 85%, from a point 126 just distal of the termination of tubular body 118 to a second, more distal point 123 on extending portion 124. For example, where a core wire end 122 has a cross-sectional area of about 0,002 mm² (0.00003 in²) and is inserted into a catheter tubular body having a lumen with an inner diameter of about 0,2 mm (0.0093"), core wire 120 preferably tapers from a cross-sectional area of about 0,2 mm² (0.0003 in²) at point 126 to about 0,003 mm² (0.0000049 in²) at point 123. A region of constant cross-sectional area 129 may be provided to core wire 120 at points distal to portion 123. In this and other embodiments, catheter tubular body 118 may have varying wall thickness, as described above.

As illustrated in Figure 4, an inflatable balloon 160 is mounted on tubular body 118. Balloon 160 has a proximal portion 162 and a distal portion 164. Proximal portion 162 and distal portion 164 are both secured to the outer surface of tubular body 118. Balloon 160 may be secured to tubular body 118 by any means known to those of skill in the art, such as adhesives or heat bonding. In one preferred embodiment, balloon 160 is a compliant balloon formed out of a material comprising a block copolymer of styrene-ethylene-butadiene-styrene, as described below. Tapers 166 may be provided proximally and distally of balloon 160.

Core wire 120 may be provided with a bend 125, such that core wire 120 bends back upon itself to form portions 127a and 127b, as shown in Figure 4. Bend 125 and portions 127a and 127b facilitate shaping of the distal extremity of a guidewire incorporating core wire 120 during its use. In one preferred embodiment, core wire portions 127a and 127b are of approximately the same length. Bend 125 is secured within a hemispherical solder bump or protrusion 150 which is carried by the distal extremity of a coil 180 formed of a suitable radiopaque material such as gold, platinum or a platinum alloy. Coil 180 can have a suitable outside diameter which corresponds to the outer diameter of tubular body 118, and can have a suitable length ranging from about 2 to about 10 cm. For example, where tubular body 118 has an outer diameter of 0,35 mm (0.014 inches), and core wire 120 has a length of 37 mm, coil 180 may have a length of about 35 mm.

Coil 180 is secured to the distal end of tubular body 118 by suitable means such as an adhesive or by soldering or brazing. One preferred adhesive type for connecting coil 180 to tubular body 118 is cyanoacrylate, although, as will be appreciated by those of skill in the art, other similar adhesives adopted to form metal to metal bonds may also be used.

Expandable members, such as balloons used on catheters incorporating the core wire may be formed out of any material used to manufacture inflatable catheter balloons, such as latex, silicone, or inelastic materials, such as polyethylene terephtalate, or combinations of material comprising a block copolymer of styrene-ethylene-butylene-styrene (SEBS). It has been found that SEBS resins can be used to form catheter balloons with improved elasticity in comparison to other compliant balloon materials. Preferred SEBS resins for balloons may be purchased under the trade name C-FLEX, sold by Consolidated Polymer Technologies. In particular, the C-FLEX (TM) resin grade R70-050-000 is presently preferred.

As a first step in the balloon formation process, the selected SEBS resin is extruded to form a tube which will subsequently be shaped into a balloon. The resin may be extruded to form tubes having a variety of different internal and outer diameters, as can be readily appreciated by those of skill in the art. It is preferable, however, that the inner diameter of the extruded tubing be no more than about 120% greater and preferably no more than about 80% greater than the outer diameter of the catheter tubular body to which the finished balloon will be mounted. For example, where the outer diameter of tubular body 18 is about 0,35 mm (0.014 inches), as is preferable for many hollow guidewire applications, the inner diameter of this extruded SEBS tubing is preferably from about 0,4 mm (.016 inches) to about 0,75 mm (.030 inches), more preferably 0,5 mm to about 0,7 mm (.020 inches to about .027 inches), and optimally about 0,63 mm (.025 inches). The outer diameter of the extruded SEBS tube is preferably about 0,9 to 1,5 mm (.035 inches to about .060 inches), more preferably 1 to 1,5 (.042 inches to about .058 inches), and optimally is 1,35 mm (.053 inches). (For a 3.5 - 4.5 mm balloon.)

Any suitable one inch extrusion apparatus may be used to form the extruded SEBS tubes. For example, balloons may be formed from tubing extruded on a 0,25 mm (1") Harrel extruder, set to a draw down ratio of from about 1 to about 1.4, more preferably to a draw down ratio of about 1 to about 1.2.

It is important to monitor the extrusion process to ensure that the resulting tubing has substantially uniform inner and outer diameters along its length. In other words, uniform concentricity of the resulting extruded tube is very important. One important variable that needs to be monitored and controlled is the amount of tension which is applied to the tubing during the extrusion process. It is important not to apply too much tension, so that the tubing keeps proper dimensions along its length. For example, for extrusion of tubing having an inner diameter of about 0.025 inches and an outer diameter of about 1,35 mm (0.053 inches) applied tension during extension preferably does not exceed 0,11 kg (4 oz).

Extrusion tension can be controlled by a variety of means, as is known to those of skill in the art. For example, extrusion tension can be controlled by using hand extrusion, by low tension pullers, by low tension winders, or by other means known to those of skill in the art.

The extruded SEBS tubing has an inner diameter much larger than the outer diameter of the catheter tubular body, such that the tubing may not be directly mounted to the tubular body to form a balloon. Accordingly, the inner diameter of the SEBS tubing must be reduced before the SEBS tube may be mounted to the catheter tubular body as a balloon.

Thus, one important step in forming the balloons involves reducing both the inner and outer diameter of the SEBS tubes by a pre-stretching process. Advantageously, the pre-stretching process not only reduces the inner and outer diameters such that the SEBS tubing may be mounted to a catheter tubular body as a balloon, but also results in a finished compliant balloon which exhibits reduced longitudinal expansion upon inflation, indeed, it has been discovered that the pre-stretching process is capable of reducing longitudinal expansion of finished SEBS balloons by from about 20% to about 50%.

The pre-stretching process generally comprises longitudinally stretching the extruded SEBS tube by at least 200%, such that substantially all lengthwise deformation of the SEBS tube occurs along a line parallel to the longitudinal axis of the SEBS tube. In other words, the tube is stretched lengthwise while controlling the stretching process variables to minimize curvature or other bends in the tube. Preferably, the extruded SEBS tube is stretched by at least 400%, more preferably by at least 600%, and optimally by at least 900%, such that the inner diameter of the SEBS tube decreases from its starting size to about 0,05-0,075 mm (0.002-0.003 inches) greater than the outer diameter of the catheter tubular body to which the extruded tube is to be mounted as a balloon. Furthermore, the pre-stretching process also preferably reduces the outer diameter of the SEBS tube from its starting size, to an outer diameter which is at least 15% smaller, more preferably 25% smaller, and optimally at least 30% smaller than the starting outer diameter size. For example, where the starting inner diameter of an extruded SEBS tube is about 0,06 mm (0.025 inches), and the starting outer diameter of the tube is 0,35 mm (0.053 inches) the tube may be stretched so that it length increases by about 600-700%, so that the resulting inner diameter of the tube is about 0,4 mm (0.016 inches) and the resulting outer diameter is about 0,9 mm (0.035 inches). A stretched tube with these dimensions is preferably mounted to the embodiment of the tubular body 18 having an outer diameter of about 0,35 mm (0.014 inches) to form a balloon.

As is readily appreciated by those of skill in the art, where the outer diameter of the tube is reduced more than the inner diameter, the thickness of the tube also decreases. Preferably the thickness is reduced by at least 10%, more preferably by at least 20%, and optimally by at least 30%. Greater reductions in thickness may also result from the pre-stretching process and still function, depending upon the grade of SEBS resin and the stretching conditions used. The manner of adapting these different resin grades and stretching conditions will be apparent to those of skill in the art in view of the description herein.

The pre-stretching process is preferably carried out at temperature which facilitates the stretching without contributing to any undesirable bending of the tube. For most grades of SEBS, temperatures of between 0° to about 90°C are preferred. Temperatures lower than this generally require the application of increased longitudinal force to carry out the stretching process, resulting in increased risk of nonuniform stretching or bending of the resulting tube. Moreover, at temperatures greater than about 90°C, the SEBS block copolymer used to form the tubing tends to soften considerably, such that gravitational force may introduce unwanted bend or curvature in the tube. Optimally, stretching is done at about 25-30°C.

The stretching rate also has an important effect on the properties of the resulting balloon. Preferably, the SEBS tubing is stretched at a rate of from about 0.5 cm per min to about 50 cm per minute, more preferably at a rate of less than 30 cm/min., and optimally is stretched at a rate of 10 cm inches per minute at room temperature. Stretching rates greater than the maximum amount may result in undesirable residual elongation. After the pre-stretching process is completed, the stretched SEBS tubing is preferably permitted to settle for a period of about 10-15 seconds, prior to removal from the stretching apparatus.

Once the pre-stretching process is completed, the stretched tubing is preferably cut to appropriate balloon length within two hours of the stretching, otherwise tube relaxation may occur which adversely affects the dimensions of the stretched tube. Cutting may be performed by any means known to those of skill in the art. One preferred cutting process comprises inserting a stainless steel mandrel into a polyamide tube, and then inserting the mandrel/polyamide tube combination into the lumen of the stretched SEBS tube. The stainless steel mandrel is then removed, leaving the polyamide tube within the stretched SEBS tube. The polyamide tube provides structural support to the SEBS tube during the cutting process, facilitating the formation of straight cut edges. For example, for a stretched SEBS tube having an inner diameter of about 0,4 mm (0.016 inches) and an outer diameter of about 0,9 mm (0.035 inches), a stainless steel mandrel having an outer diameter of 0,33 mm (0.013 inches) is inserted into a polyamide tube having an inner diameter of 0,37 mm (0.0145 inches) and an outer diameter of 0,04 mm (0.0155 inches). The combination is then inserted into the stretched SEBS tube, and the stainless steel mandrel is removed. A standard cutting tool, such as a razor blade is then used to cut through the SEBS tubing and the polyamide tubing into segments having lengths of approximately 9 mm. After the cutting is competed, the polyamide tubing is removed.

The stretched and cut pieces of SEBS tubing may then be bonded to catheter tubular bodies to form compliant inflatable balloons. Conventional balloon bonding techniques may be used to mount the SEBS balloons to catheter tubular bodies. Such techniques include adhesive bonding and heat bonding, as known to those of skill in the art. In one preferred embodiment where the catheter tubular body comprises nitinol, a primer is first applied to the inner surface of each end of the SEBS tube to improve the bonding of the SEBS tube to nitinol. One suitable primer found useful for the priming step is 7701 LOCTITE, sold by Loctite Corp. However, as will be appreciated by those of skill in the art, other primers may also be used. The primer is preferably applied only to the inner surface of the SEBS tube at its ends, and more preferably, each end inner surface is primed for a distance of about 2 mm extending inward.

After the priming step, the primed tubing is slid over the catheter tubular body to the appropriate balloon position, such as over a fill hole in communication with an inflation lumen. Each end of the SEBS tubing is then mounted to the catheter tubular body to form a fluid tight seal. In a preferred embodiment, a cyanoacrylate adhesive is used to bond the SEBS tubing to the nitinol catheter tubular body. One preferred cyanoacrylate is LOCTITE 4011, sold by Loctite Corp. When using the LOCTITE 4011 adhesive, however, it is important to control the humidity of the surrounding environment, such that the humidity is maintained at at least 35% to 40%.

While adhesive bonding is taking place, clamps are preferably placed adjacent to the working area of the balloon to prevent adhesive flow inward. For example, if a 9 mm SEBS tube is bonded to a catheter tubular body along 2 mm at each end, clamps are placed slightly inward of the 2 mm mark, so that 5 mm of tubing is not bonded to the tubular body, and may function as a balloon.

After the SEBS tube has been bonded to the catheter tubular body to form a balloon, and the adhesive has set, tapers are preferably formed on the balloon to facilitate unhindered movement within a patient. Tapers may be added by conventional means known to those of skill in the art, such as adhesive bonding of the tapered parts separately to the catheter after the balloon has been attached. Alternately, tapers can be formed by adhesives which are applied to the balloon. In addition, it is possible to mold the balloon with a taper and then attach it.

It will be appreciated that certain variations of the core wire of the present invention may suggest themselves to those skilled in the art.

## Claims

1. A catheter comprising:
a tubular body being formed of nitinol and having a proximal end (12) and a distal end (14, 114), the tubular body (18, 118) having a lumen (40, 140) extending therethrough, and the lumen having a distal end;
a core member (120) mounted inside the lumen and having an extending portion (124) which extends past the distal end of said lumen;
an expandable member (20, 160) mounted on the distal end of the tubular body,
the expandable member having a proximal portion (162) and a distal portion (164), which portions are both mounted to the tubular body
**characterized in that**, said core member comprises a core wire secured to the distal end of the lumen.

2. A catheter as claimed in Claim 1, wherein the expandable member comprises an inflatable occlusion balloon.

3. A catheter as claimed in Claim 2, wherein the expandable member comprises a compliant balloon.

4. A catheter as claimed in any of Claims 1 to 3, wherein the length of the tubular body is from 160 to 320 cm and the length of the core wire is from 20 mm to 100 mm.

5. A catheter as claimed in Claim 4 wherein the core wire has a length of 25 mm to 50 mm.

6. A catheter as claimed in any of Claims 1 to 5, further comprising a coil (180) secured to the distal end of the tubular body, said core wire extending from inside said body into said coil.

7. A catheter as claimed in any of Claims 1 to 6, wherein the core wire is tapered over a length of no more than 50 mm, but at least 15 mm.

8. A catheter as claimed in any of Claims 1 to 7, wherein the core wire is tapered and has a first cross sectional area at one end of the taper and a second cross sectional area at the other end of the taper, the first cross sectional area being greater than the second cross sectional area by at least 20%.

9. A catheter as claimed in Claim 8, wherein the first cross sectional area is greater than the second cross sectional area by at least 70%.

10. A catheter as claimed any of Claims 1 to 9, wherein said extending portion has a region of constant cross sectional area.

11. A catheter as claimed in any of Claims 1 to 10, wherein the distal end of the tubular body is crimped onto the core wire to secure the core wire within the lumen.

12. A catheter as claimed in any of Claims 1 to 11, wherein the core wire comprises nitinol.

13. A catheter as claimed in any of Claims 1 to 11, wherein the core wire comprises stainless steel.

14. A catheter as claimed in any of Claims 1 to 13, wherein said tubular body comprises a guidewire.

## Patentansprüche

1. Katheter, welcher umfasst:
• einen aus Nitinol hergestellten röhrenförmigen Körper mit einem proximalen Ende (12) und mit einem distalen Ende (14, 114), wobei der röhrenförmige Körper (18, 118) ein Lumen (40, 140) aufweist, welches sich durch denselben hindurch erstreckt, und das Lumen verfügt über ein distales Ende;
• ein Kernelement (120), welches innerhalb des Lumens montiert ist und welches ein sich ausdehnendes Teil (124) aufweist, das sich über das distale Ende des besagten Lumens hinaus erstreckt;
• ein ausdehnbares Element (20, 160), welches an dem distalen Ende des röhrenförmigen Körpers montiert ist,
• wobei das ausdehnbare Element einen proximalen Teil (162) und einen distalen Teil (164) aufweist, welche beiden Teile an dem röhrenförmigen Körper montiert sind,
**dadurch gekennzeichnet, dass** das besagte Kernelement einen Kerndraht umfasst, welcher an dem distalen Ende des Lumens befestigt ist.

2. Katheter gemäß Anspruch 1, wobei das ausdehnbare Element einen aufblasbaren Ballon umfasst, der zur Okklusion dient.

3. Katheter gemäß Anspruch 2, wobei das ausdehnbare Element einen weichen Ballon umfasst.

4. Katheter gemäß irgendeinem der Ansprüche 1 bis 3, wobei die Länge des röhrenförmigen Körpers in dem Bereich von 160 bis 320 cm liegt und die Länge des Kerndrahtes in dem Bereich von 20 bis 100 mm liegt

5. Katheter gemäß Anspruch 4, wobei der Kerndraht eine Länge von 25 mm bis 50 mm aufweist.

6. Katheter gemäß irgendeinem der Ansprüche 1 bis 5, der weiterhin eine Spule (180) umfasst, die an dem distalen Ende des röhrenförmigen Körpers festgemacht ist, wobei sich der besagte Kerndraht von dem Innern des besagten Körpers in die besagte Spule hinein erstreckt.

7. Katheter gemäß irgendeinem der Ansprüche 1 bis 6, wobei der Kerndraht sich über eine Länge von nicht mehr als 50 mm, aber von mindestens 15 mm verjüngt.

8. Katheter gemäß irgendeinem der Ansprüche 1 bis 7, wobei der Kerndraht verjüngt ist und eine erste Querschnittsfläche an dem einen Ende der Verjüngung aufweist und eine zweite Querschnittsfläche an dem anderen Ende der Verjüngung, wobei die erste Querschnittsfläche um mindestens 20 % größer ist als die zweite Querschnittsfläche.

9. Katheter gemäß Anspruch 8, wobei die erste Querschnittsfläche um mindestens 70 % größer ist als die zweite Querschnittsfläche.

10. Katheter gemäß irgendeinem der Ansprüche 1 bis 9, wobei der besagte sich ausdehnende Teil einen Bereich mit einer konstanten Querschnittsfläche aufweist.

11. Katheter gemäß irgendeinem der Ansprüche 1 bis 10, wobei das distale Ende des röhrenförmigen Körpers auf dem Kerndraht gecrimpt ist, um den Kerndraht innerhalb des Lumens zu befestigen.

12. Katheter gemäß irgendeinem der Ansprüche 1 bis 11, wobei der Kerndraht Nitinol enthält.

13. Katheter gemäß irgendeinem der Ansprüche 1 bis 11, wobei der Kerndraht rostfreien Stahl enthält.

14. Katheter gemäß irgendeinem der Ansprüche 1 bis 13, wobei der besagte röhrenförmige Körper einen Führungsdraht enthält.

## Revendications

1. Cathéter comprenant :
un corps tubulaire formé de nitinol et ayant une extrémité proximale (12) et une extrémité distale (14, 114), le corps tubulaire (18, 118) ayant un lumen (40, 140) qui le traverse et le lumen ayant une extrémité distale;
un élément central (120) monté à l'intérieur du lumen et ayant une partie d'extension (124) qui s'étend au-delà de l'extrémité distale dudit lumen;
un élément expansible (20, 160) monté sur l'extrémité distale du corps tubulaire, l'élément expansible ayant une partie proximale (162) et une partie distale (164), lesdites parties étant toutes deux montées sur le corps tubulaire, **caractérisé en ce que** ledit élément central comprend un fil central fixé à l'extrémité distale du lumen.

2. Cathéter selon la revendication 1, dans lequel l'élément expansible comprend un ballonnet d'occlusion gonflable.

3. Cathéter selon la revendication 2, dans lequel l'élément expansible comprend un ballonnet souple.

4. Cathéter selon l'une quelconque des revendications 1 à 3, dans lequel la longueur du corps tubulaire se situe dans la plage de 160 à 320 cm et la longueur du fil central se situe dans la plage de 20 à 100 mm.

5. Cathéter selon la revendication 4, dans lequel le fil central a une longueur dans la plage de 25 à 50 mm.

6. Cathéter selon l'une quelconque des revendications 1 à 5, comprenant en outre une spirale (180) fixée à l'extrémité distale du corps tubulaire, ledit fil central s'étendant de l'intérieur dudit corps dans ladite spirale.

7. Cathéter selon l'une quelconque des revendications 1 à 6, dans lequel le fil central est effilé sur une longueur ne dépassant pas 50 mm, mais d'au moins 15 mm.

8. Cathéter selon l'une quelconque des revendications 1 à 7, dans lequel le fil central est effilé et a une première surface en coupe transversale à une extrémité de l'effilement et une deuxième surface en coupe transversale à l'autre extrémité de l'effilement, la première surface en coupe transversale étant plus grande que la deuxième surface en coupe transversale d'au moins 20%.

9. Cathéter selon la revendication 8, dans lequel la première surface en coupe transversale est plus grande que la deuxième surface en coupe transversale d'au moins 70%.

10. Cathéter selon l'une quelconque des revendications 1 à 9, dans lequel ladite partie d'extension a une région de surface en coupe transversale constante.

11. Cathéter selon l'une quelconque des revendications 1 à 10, dans lequel l'extrémité distale du corps tubulaire est sertie sur le fil central pour fixer le fil central à l'intérieur du lumen.

12. Cathéter selon l'une quelconque des revendications 1 à 11, dans lequel le fil central comprend du nitinol.

13. Cathéter selon l'une quelconque des revendications 1 à 11, dans lequel le fil central comprend de l'acier inoxydable.

14. Cathéter selon l'une quelconque des revendications 1 à 13, dans lequel ledit corps tubulaire comprend un fil guide.
